# EUROPEAN PATENT APPLICATION

(11) **EP 2 158 911 A1**
(43) Date of publication of application: **03.03.2010**
(21) Application number: 09177374.7
(22) Date of filing: 09.05.2006
(51) Int. Cl.: A61K 31/47, A61K 9/20, A61K 9/28, A61P 11/06

(54) **Stable pharmaceutical formulations of montelukast sodium**

(30) Priority: 09.02.2006 US 772258 P
(62) Divisional of application: 06252449.1
(71) Applicant: Teva Pharmaceutical Industries Ltd, 49131 Petah Tiqva (IL)
(72) Inventor: Hrakovsky, Julia, Rosh Ha-Ayin 48057 (IL); Tenengauzer, Ruth, Hod Hasharon 45322 (IL); Bogomolny, Grigory, Kefar-Sava 44391 (IL); Dolitsky, Yehudit, Petah Tiqva 49651 (IL)
(74) Representative: Russell, Tim

(57) **Abstract**

The invention encompasses stable pharmaceutical compositions comprising montelukast or salts thereof and methods of preparing the same.

## Description

### FIELD OF THE INVENTION

The invention encompasses stable pharmaceutical compositions comprising montelukast or salts thereof and methods of preparing the same. Preferably, the salt is the sodium salt. In particular, the invention encompasses pharmaceutical compositions in the form of film coated tablets and chewable tablets.

### BACKGROUND OF THE INVENTION

Montelukast is apparently a selective, orally active leukotriene receptor antagonist that inhibits the cysteinyl leukotriene CysLT₁ receptor.

The chemical name for montelukast sodium is [R-(*E*)]-1-[[[1-[3-[2-(7-chloro-2-quinolinyl)ethenyl]phenyl]-3-[2-(1-hydroxy-1-methylethyl)phenyl]propyl]thio]methyl] cyclopropaneacetic acid, monosodium salt. Montelukast sodium salt is understood to be represented by the following structural formula:

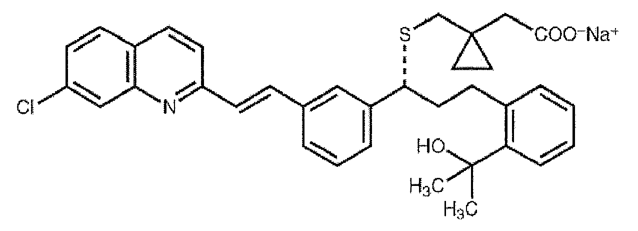

U.S. patent No. 5,565,473 ("'473 patent") is listed in the FDA's Orange Book for montelukast sodium. The '473 patent recites a broad class of leulcotriene antagonists as "anti-asthmatic, anti-allergic, anti-inflammatory, and cycloprotective agents" represented by a generic chemical formula. '473 patent, col. 2,1. 3 to col. 4,1. 4. Montelukast is among the many compounds represented by that formula. The '473 patent also refers to pharmaceutical compositions of the class of leukotriene antagonists of that formula with pharmaceutically acceptable carriers. *Id*. at col. 10,11. 42-46.

Montelukast sodium is currently marketed by Merck in the form of film coated tablets and chewable tablets under the trade name Singular^{®}. The film coated tablets reportedly contain montelukast sodium and the following inactive ingredients: microcrystalline cellulose, lactose monohydrate, croscarmellose sodium, hydroxypropylcellulose, magnesium stearate, titanium dioxide, red ferric oxide, yellow ferric oxide, and carnauba wax. The chewable tablets reportedly contain montelukast sodium and the following inactive ingredients: mannitol, microcrystalline cellulose, hydroxypropylcellulose, red ferric oxide, croscarmellose sodium, cherry flavor, aspartame, and magnesium stearate. Physicians' Desk Reference, 59th ed. (2005), p. 2141.

However, there is a need in the art to improve the stability of compositions of montelukast and particularly those of the sodium salt.

### SUMMARY OF THE INVENTION

One embodiment of the invention encompasses a stable pharmaceutical composition comprising montelukast or a salt thereof and a pharmaceutically acceptable excipient selected from at least one of diluent, binder, disintegrant, wetting agent, lubricant, and glidant, provided that the pharmaceutically acceptable excipient is not microcrystalline cellulose.

Preferably, the salt is montelukast sodium.

In an embodiment of the invention the pharmaceutical composition contains montelukast sodium and also contains the corresponding montelukast sulfoxide of formula (I): 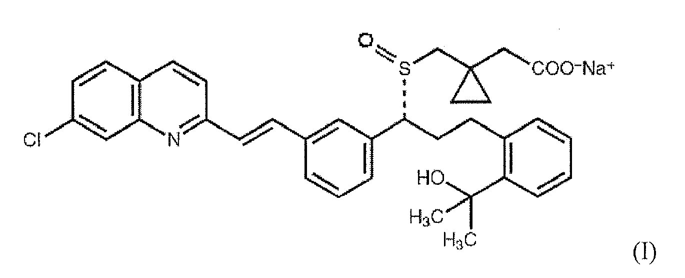 wherein the corresponding montelukast sulfoxide of formula (I) in the composition does not increase by more than 1% by weight from the initial amount of montelukast after storage at about 40°C and about 75% relative humidity for 3 months.

Preferably, the sulfoxide content has not increased by more than 0.5% by weight of the initial amount of montelukast after storage at about 40°C at about 75% relative humidity for 3 months. More preferably, the sulfoxide content has not increased by more than 0.3% by weight of the initial amount of montelukast after storage at about 40°C at about 75% relative humidity for 3 months. Most preferably, the sulfoxide content has not increased by more than 0.1% by weight of the initial amount of montelukast after storage at about 40°C at about 75% relative humidity for 3 months.

In another embodiment of the invention, the pharmaceutical composition comprises montelukast sodium.

In another embodiment of the invention, immediately after preparation of the pharmaceutical composition, the corresponding sulfoxide is present in an amount of not more than 0.2% by weight of montelukast in the pharmaceutical composition.

In another embodiment of the invention, the stable compositions of the invention encompass solid pharmaceutical dosage forms. Preferably, the solid pharmaceutical dosage forms are film coated tablets or chewable tablets.

Film coated tablets may comprise the pharmaceutical composition and a coating agent, provided that the coating agent is not microcrystalline cellulose. Preferably, the film coated tablets comprise montelukast sodium, lactose monohydrate, hydroxypropylcellulose, starch, sodium starch glycolate, magnesium stearate, and a coating. The coating may be made from a commercially available powder mix for preparing coating suspensions such as Opadry^{®}. Opadry^{®}, available from Colorcon, has hydroxypropyl cellulose, hypromellose, titanium dioxide, and iron oxide. More preferably, the film coated tablet comprises about 5% by weight montelukast sodium, about 62% by weight lactose, about 2% by weight hydroxypropylcellulose, about 18% by weight starch, about 9% by weight sodium starch glycolate, about 1% by weight magnesium stearate, and about 3% by weight Opadry^{®}. The ordinarily skilled artisan will recognize that the coating can be prepared from the constituent elements rather than the commercially available premixed preparation.

The chewable tablets of the invention may comprise the pharmaceutical composition and at least one of a sweetening agent, flavoring agent, or coloring agent, provided that the tablet does not contain microcrystalline cellulose. Preferably, the chewable tablet comprises montelukast sodium, hydroxypropylcellulose, sodium starch glycolate, mannitol, coloring agent (e.g., iron oxide), additional sweetening agent such as aspartame, flavoring agent, and magnesium stearate. More preferably, the chewable tablet comprises about 2% by weight montelukast sodium, about 2% by weight hydroxypropylcellulose, about 5% by weight sodium starch glycolate, about 87% by weight mannitol, about 0.5% by weight color iron oxide, about 0.5% by weight aspartame, about 2% by weight flavoring agent, and about 1% by weight magnesium stearate.

### DETAILED DESCRIPTION

Montelukast compositions are subject to degradation during manufacture and storage. It is believed that the montelukast degrades into its corresponding sulfoxide. The sulfoxide is an inactive impurity, which reduces the effective dosage of montelukast when it is administered to a patient. The present invention overcomes this problem by providing compositions of montelukast that are stable to this degradation.

As used herein, unless otherwise defined, the term "corresponding sulfoxide" refers to montelukast or a salt thereof wherein the sulfide group in the β-position relative to the cyclopropane group has been oxidized to a sulfoxide group, e.g. the sulfoxide of formula (I) wherein the montelukast salt is montelukast sodium has the formula:

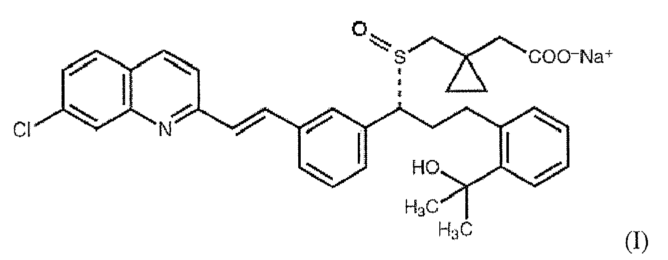

As used herein with respect to pharmaceutical compositions, unless otherwise defined, the term "stable" means that the amount of the corresponding sulfoxide within the montelukast in the packaged pharmaceutical composition has not increased by more than 1% by weight from the initial amount of montelukast after storage at about 40°C and about 75% relative humidity for 3 months. Preferably, the corresponding sulfoxide content has not increased by more than 0.5% by weight of the initial amount of montelukast after storage at about 40°C at about 75% relative humidity for 3 months. More preferably, the corresponding sulfoxide content has not increased by more than 0.3% by weight of the initial amount of montelukast after storage at about 40°C at about 75% relative humidity for 3 months. Most preferably, the corresponding sulfoxide content has not increased by more than 0.1% by weight of the initial amount of montelukast after storage at about 40°C at about 75% relative humidity for 3 months.

As used herein, unless otherwise defined, the term "accelerated storage conditions" refers to storage of montelukast at about 40°C at about 75% relative humidity for 3 months.

As used herein unless otherwise defined, the term "immediately after preparation," as applied to formulations, means the time elapsed from the preparation of the formulation and not exceeding 48 hours.

Comparative testing with montelukast sodium was performed to determine the conditions which cause the degradation of montelukast sodium into the corresponding sulfoxide. Compositions of montelukast sodium and each of the excipients of the prior art tablets were prepared and subjected to stressed storage conditions. The amount of the corresponding sulfoxide present in each of the compositions was measured by high performance liquid chromatography ("HPLC") both before and after storage. It was found that the amount of the corresponding sulfoxide in the composition increased by over 250% in the presence of microcrystalline cellulose over the storage period.

Not to be limited by theory, it is believed that the poor stability of the prior art compositions of montelukast can be attributed to the presence of microcrystalline cellulose. Microcrystalline cellulose may contain peroxide, which can catalyze the conversion of montelukast to its corresponding sulfoxide. For example, montelukast sodium may degrade into the sulfoxide of formula (I).

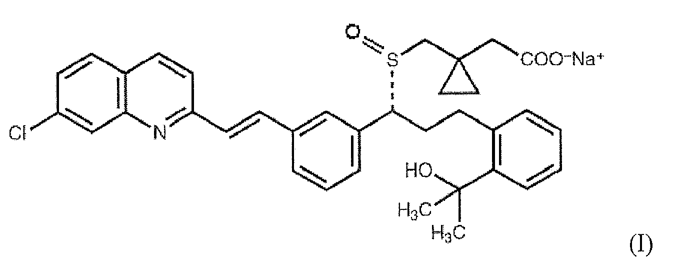

One embodiment of the invention encompasses stable pharmaceutical compositions comprising montelukast or a salt thereof and a pharmaceutically acceptable excipient selected from at least one of diluent, binder, disintegrant, wetting agent or lubricant, provided that the pharmaceutically acceptable excipient is not microcrystalline cellulose. Optionally, the pharmaceutical compositions of the invention further comprise at least one coating agent, sweetening agent, flavoring agent, coloring agent, or glidant.

Diluents increase the bulk of a solid pharmaceutical composition, and may make a pharmaceutical dosage form containing the composition easier for the patient and care giver to handle. Diluents used in the composition include diluents commonly used in solid pharmaceutical compositions. Diluents include, but are not limited to, calcium carbonate, calcium phosphate (dibasic or tribasic), calcium sulfate, dextrates, dextrin, dextrose excipient, fructose, kaolin, lactitol, anhydrous lactose, lactose monohydrate, maltose, mannitol, sorbitol, sucrose, starch, pregelatinized starch, or talc. Preferably, the diluent is at least one of lactose monohydrate, starch, or mannitol. Typically, the diluent is present in an amount of about 60% to about 95% by weight of the composition.

Binders help to bind the active ingredient and other excipients together. Binders used in the composition include binders commonly used in solid pharmaceutical compositions. Binders include, but are not limited to, acacia, alginic acid, carbomer, sodium carboxymethylcellulose, dextrin, ethylcellulose, gelatin, glucose, guar gum, hydroxypropylcellulose, maltose, methylcellulose, povidone, starch, methylcellulose, or polyethylene oxide. Preferably, the binder is hydroxypropylcellulose. Typically, the binder is present in an amount of about 1% to about 5% by weight of the composition.

Disintegrants increase the dissolution rate of a solid pharmaceutical composition in the patient's body. Disintegrants used in the composition include disintegrants commonly used in solid pharmaceutical compositions. Disintegrants include, but are not limited to, alginic acid, croscarmellose sodium, crospovidone, potassium polacrilin, sodium starch glycolate, and starch. Preferably, the disintegrant is at least one of sodium starch glycolate or starch. Typically, the disintegrant is present in an amount of about 5% to about 15% by weight of the composition.

Wetting agents are added to pharmaceutical compositions for facilitating processing. Wetting agents used in the composition include wetting agents commonly used in solid pharmaceutical compositions. Wetting agents used in the composition include, but are not limited to, sodium lauryl sulfate. Generally, suitable wetting agents can be selected by the method outlined in example 1 below, to ensure that they have no excessive adverse effect on the stability of the montelukast.

Lubricants are added to a pharmaceutical composition for ease in processing, to prevent adhesion to the equipment used during processing. Lubricants used in the composition include lubricants commonly used in solid pharmaceutical compositions. Lubricants used in the composition include, but are not limited to, calcium stearate, glyceryl behenate, magnesium stearate, mineral oil, polyethylene glycol, sodium stearyl fumarate, stearic acid, talc, vegetable oil, sodium lauryl sulfate, or zinc stearate. Preferably, the lubricant is magnesium stearate. Typically, the lubricant is present in an amount of about 0.5% to about 2% by weight of the composition.

Coating agents facilitate the administration of a solid pharmaceutical composition to a patient, by making it easier for a patient to swallow the composition. Coating agents used in the composition include coating agents commonly used in solid pharmaceutical compositions. Coating agents include, but are not limited to, sodium carboxymethylcellulose, cellulose acetate, cellulose acetate, phthalate, ethylcellulose, gelatin, pharmaceutical glaze, hydroxypropylcellulose, hydroxypropylmethylcellulose, hypromellose phthalate, methacrylic acid copolymer, methylcellulose, polyethylene glycol, polyvinyl acetate phthalate, shellac, sucrose, titanium dioxide, lactose, or carnauba wax. The coating may also contain a coloring agent. Preferably, the coating agent is Opadry^{®}, which is a commercially available coating material prepared by Colorcon and contains hydroxypropyl cellulose, hypromellose, titanium dioxide, and iron oxide. The ordinary practitioner will recognize that this coating agent may be prepared from these ingredients rather than the commercially available premixed Opadry^{®} preparation, without departing from the scope of the invention. Typically, the coating agent is present in an amount of about 1% to about 3% by weight of the composition.

Sweetening agents are used to sweeten pharmaceutical compositions. Sweetening agents used in the composition include sweetening agents commonly used in solid pharmaceutical compositions. Sweetening agents include, but are not limited to, aspartame, dextrates, dextrose, fructose, mannitol, saccharin, sorbitol, sucralose, sucrose, sugar, or syrup. Preferably, the sweetening agent is at least one of aspartame or mannitol. When aspartame is used, care must be taken to use a minimal amount so as not to effect an interaction with the active ingredient in the chewable tablets of the invention; hence the amount should be about 1 mg per tablet or less. Typically, the sweetening agent is present in an amount of about 0.5% to about 90% by weight of the composition.

Flavoring agents make a pharmaceutical composition more palatable to the patient. Flavoring agents used in the composition include flavoring agents commonly used in solid pharmaceutical compositions. Flavoring agents used in the composition include, but are not limited to, maltol, vanillin, ethyl vanillin, menthol, citric acid, fumaric acid, ethyl maltol, tartaric acid, peppermint, artificial or natural fruit flavors. Typically, the flavoring agent is present in an amount of about 1% to about 3% by weight of the composition.

Coloring agents improve the appearance of a pharmaceutical composition and/or facilitate patient identification of the composition. Coloring agents used in the composition include coloring agents commonly used in solid pharmaceutical compositions. Coloring agents used in the composition include, but are not limited to, caramel, ferric oxides (red, yellow, or black), or natural or synthetic organic colors and lakes. Preferably, the coloring agent is ferric oxide. Typically, the coloring agent is present in an amount of about 0.1% to about 1% by weight of the composition.

Glidants improve the flowability of a non-compacted solid composition and improve the accuracy of dosing. Glidants used in the composition include glidants commonly used in solid pharmaceutical compositions. Glidants used in the composition include, but are not limited to, colloidal silicon dioxide, magnesium trisilicate, starch, talc, or tribasic calcium phosphate. Preferably, the glidant is colloidal silicon dioxide. Typically, the glidant is present in an amount of about 0.3% to about 1.5% by weight of the composition.

In one preferred embodiment of the invention, the pharmaceutical compositions comprise montelukast sodium and a pharmaceutically acceptable excipient selected from at least one of diluent, binder, disintegrant, or lubricant, provided that the pharmaceutically acceptable excipient is not microcrystalline cellulose. Optionally, the pharmaceutical compositions further comprise at least one coating agent, sweetening agent, flavoring agent, coloring agent, or glidant.

In another embodiment of the invention, the pharmaceutical compositions are formulated into solid pharmaceutical dosage forms. Solid pharmaceutical dosage forms include those commonly known to those of ordinary skill in the art. Solid pharmaceutical dosage forms include, but are not limited to, tablets, capsules, powders, granules, suppositories, sachets, or troches. Preferably, the solid pharmaceutical dosage form is a tablet or capsule. More preferably, the solid pharmaceutical dosage form is a tablet.

In one preferred embodiment of the invention, the solid pharmaceutical dosage form is a film coated tablet. Preferably, the film coated tablet comprises montelukast sodium, diluent, binder, disintegrant, lubricant, and coating agent, with the proviso that the tablet does not comprise microcrystalline cellulose. More preferably, the film coated tablet comprises montelukast sodium, lactose monohydrate, hydroxypropylcellulose, starch, sodium starch glycolate, magnesium stearate, and Opadry^{®}. Most preferably, the film coated tablet comprises about 5% by weight montelukast sodium, about 62% by weight lactose monohydrate, about 2% by weight hydroxypropylcellulose, about 18% by weight starch, about 9% by weight sodium starch glycolate, about 1% by weight magnesium stearate, and about 3% by weight Opadry^{®}.

In another preferred embodiment of the invention, the solid pharmaceutical dosage form is a chewable tablet. Preferably, the chewable tablet comprises montelukast sodium, diluent, binder, disintegrant, lubricant, flavoring agent, sweetening agent, and coloring agent, with the proviso that the tablet does not comprise microcrystalline cellulose. More preferably, the chewable tablet comprises montelukast sodium, hydroxypropylcellulose, sodium starch glycolate, mannitol, color iron oxide, aspartame, flavoring agents, and magnesium stearate. Most preferably, the chewable tablet comprises about 2% by weight montelukast sodium, about 2% by weight hydroxypropylcellulose, about 5% by weight sodium starch glycolate, about 87% by weight mannitol, about 0.5% by weight color iron oxide, about 0.5% by weight aspartame, about 2% by weight flavor, and about 1% by weight magnesium stearate.

Although the use of the suggested excipients should result in stable compositions, there may be variability within the commercially available grades and types of excipients, and impurities that might be present in an excipient from a particular source. A test protocol similar to that described in Example 1 can determine if a particular excipient is a source for instability.

The solid pharmaceutical dosage forms of the invention may be prepared by conventional processes known to those of ordinary skill in the art, including, but not limited to, wet granulation, dry granulation such as slugging or compaction, or direct compression of the formulation into tablets or filling into capsules. Preparation techniques not involving wet granulation are preferred from the point of view of stability, although considerations as to the physical properties of the finished tablet may mandate the use of wet granulation. Where wet granulation is used, the careful choice of excipients is particularly important.

Typically, the film coated tablets are prepared by dry blending. For example, the blended composition of the active ingredients and excipients may be compacted into a slug or a sheet and then comminuted into compacted granules. The compacted granules may subsequently be compressed into a tablet, typically with the addition of a lubricant.

Preferably, montelukast sodium is blended with diluents and binders to form a blend. Disintegrant is then added to the blend and blended. Lubricant is then added to the blend and blended. The blend is compressed into a tablets and coated with coating agent to form film coated tablets.

Typically, the chewable tablets are prepared by wet granulation. In wet granulation, some or all of the active ingredients and excipients in powder form are blended and then further mixed in the presence of a liquid, typically water, that causes the powders to clump into granules. The granulate can be screened and/or milled, dried and then screened and/or milled to the desired particle size. The dried granulate may then be tabletted, or other excipients may be added prior to tableting.

Preferably, montelukast sodium, binder, disintegrant, diluent, sweetening agent, flavoring agent, and coloring agent are granulated using purified water as a granulating liquid to form a granulate. The granulate is then dried, milled, and blended with lubricant to form a blend. The blend is then compressed into a chewable tablet.

Having described the invention with reference to certain preferred embodiments, other embodiments will become apparent to one skilled in the art from consideration of the specification. The invention is further defined by reference to the following examples describing in detail methods for the preparation and testing of the montelukast pharmaceutical compositions. It will be apparent to those skilled in the art that many modifications, both to materials and methods, may be practiced without departing from the scope of the invention.

### EXAMPLES

### Example 1:

Sample mixtures of montelukast sodium and each of the individual excipients were prepared. The composition of each of the samples is listed in Tables 1a and 1b. Each sample was stored at 55°C for 48 hours. The percentage by weight of sulfoxide of formula (I) relative to montelukast sodium in each sample was measured at 0 hours and at 48 hours by HPLC. HPLC was performed on a Beta-Basic C18 analytical column (150x4.6 mm I.D.), packed with 5µm diameter particles (Thermo Election Corporation). The mobile phase was a mixture of acetonitrile and 20 mM KH₂PO₄ (60:40) and its flow-rate was 1.5 mL/min. The UV detector was set at 225 nm or 281 nm and the column temperature was 40°C. The results are shown in Table 1.

**Table 1: Analysis of Montelukast Sodium Compositions with Various Excipients**

| Sample | Composition | % Sulfoxide of Formula (I) | |
|---|---|---|---|
| | | 0 hours | 48 hours |
| 1 | Montelukast sodium (a) | 0.63 | - |
| 2 | Montelukast sodium (1 g) / microcrystalline cellulose (10.1 g) | 0.59 | 1.52 |
| 3 | Montelukast sodium (1.5 g) / lactose (9.5 g) | 0.60 | 0.63 |
| 4 | Montelukast sodium (3.5 g) / hydroxypropylcellulose (7.4 g) | 0.58 | 0.62 |
| 5 | Montelukast sodium (7.5 g) / crospovidone (3.6 g) | 0.69 | 0.68 |
| 6 | Montelukast sodium (9.4 g) / magnesium stearate (1.8 g) | 0.56 | 0.57 |
| 7 | Montelukast sodium (b) | 0.26 | - |
| 8 | Montelukast sodium (1 g) / mannitol (36 g) | 0.25 | 0.28 |
| 9 | Montelukast sodium (2 g) / aspartame (2 g) | 0.24 | 0.73 |
| 10 | Montelukast sodium (2 g) / aerosol (2 g) | 0.25 | 0.25 |
| 11 | Montelukast sodium (2 g) / microcrystalline cellulose (20 g) / crospovidone (8 g) | 0.74 | 1.2 |

As illustrated by Table 1, the presence of microcrystalline cellulose in the composition caused a substantial increase in the amount of the sulfoxide of formula (I) upon storage. The amount of sulfoxide of formula (I) also increased in the presence of aspartame, although from a taste perspective, it may have to included in the chewable tablets at a low level. Typically, aspartame should be present in an amount of not more than about 1 mg per tablet in order to achieve the desired stability. One may also be able to substitute mannitol for aspartame to improve stability, while preserving taste. There was no substantial change in the amount of the sulfoxide of formula (I) upon storage in the presence of the other excipients.

### Example 2:

A pharmaceutical composition of montelukast sodium 10 mg tablets was prepared by a wet granulation method. Montelukast sodium (39.52 g), hydroxypropyl cellulose (15.2 g), crospovidone (76.0 g) and lactose (659.68 g) was mixed. The mixture was then granulated using purified water as a granulating liquid to form a granulate. The granulate was dried, milled and blended with magnesium stearate (7.6 g) to form a final blend. The final blend was compressed into the 10 mg tablets.

### Example 3:

A pharmaceutical composition of montelukast sodium was prepared by a dry mix method. A mixture was made of montelukast sodium (1352 g), lactose monohydrate (17303 g), and hydroxypropylcellulose (520 g). The mixture was blended for 20 minutes to form a blend. Starch (5200 g) and sodium starch glycolate (2600 g) were then added to the blend and blended for 10 minutes. Subsequently, magnesium stearate (325 g) was added to the blend and blended for an additional 5 minutes. The blend was compressed into tablets. The tablets were film coated using Opadry^{®} (780 g).

### Example 4:

A pharmaceutical composition of montelukast sodium was prepared by a dry mix method. A mixture was made of montelukast sodium (52 g), lactose monohydrate (634 g), and hydroxypropylcellulose (20 g). The mixture was blended for 20 minutes to form a blend. Starch (200 g), sodium lauryl sulfate (31.5g) and sodium starch glycolate (100 g) were then added to the blend and blended for 10 minutes. Subsequently, magnesium stearate (12.5 g) was added to the blend and blended for an additional 5 minutes. The blend was compressed into tablets.

| Composition | % Sulfoxide of Formula (I) | |
|---|---|---|
| | 0 hours | 72 hours at 55 degrees |
| Example 4 | 0.1 | 0.1 |

### Example 5:

A pharmaceutical composition of montelukast sodium chewable tablets was prepared by wet granulation. A mixture was made of montelukast sodium (41.6 g), hydroxypropyl cellulose (40 g), sodium starch glycolate (80 g), mannitol (1490.4 g), color iron oxide (4 g), aspartame (8 g), and flavor (32 g). The mixture was then granulated using purified water as a granulating liquid to form a granulate. The granulate was dried, milled and blended with magnesium stearate (24 g) to form a blend. The blend was compressed into chewable tablets.

### Example 6:

A pharmaceutical composition of montelukast sodium chewable tablets was prepared by a dry mix direct compression method. A mixture was made of montelukast sodium (23.4 g), hydroxypropylcellulose (22.5 g), sodium starch glycolate (45 g), aspartame (4.5 g), color (4.5 g), mannitol (858.6 g) and cherry flavor (18 g) and the mixture was blended for 15 minutes. Subsequently, magnesium stearate (13.5 g) was added to the blend and blended for an additional 5 minutes. The blend was compressed into chewable tablets.

### Example 7:

The pharmaceutical compositions prepared in Examples 3 and 4 were exposed to accelerated storage conditions, i.e., storage at about 40°C and about 75% relative humidity. The percentage by weight of sulfoxide of formula (I) relative to montelukast sodium in each sample was measured by HPLC immediately after the compositions were prepared and after 1, 2 and 3 months under accelerated storage conditions. HPLC was performed on a YMC-Pack ODS-AQ analytical column (100x4.6 mm I.D.), packed with 3µm diameter particles (YMC SEPARATION TECHNOLOGY). The mobile phase was a mixture of acetonitrile and 20 mM KH₂PO₄ at pH 2.0 (60:40) and its flow-rate was 1.0 mL/min. The UV detector was set at 285 nm and the column temperature was 30°C. Samples of Singular^{®} were also analyzed under the same conditions. The results are shown in Table 2.

**Table 2: Stability of Montelukast Sodium Compositions Under Accelerated Storage Conditions**

| Sample | % Sulfoxide of Formula (I) | | | |
|---|---|---|---|---|
| | Initial | 1 month | 2 months | 3 months |
| Composition of Example 3 | <0.1% | 0.1% | <0.1% | <0.1% |
| Composition of Example 5 | 0.1% | 0.2% | 0.2% | - |
| Singular^{®}, 10 mg tablet | 0.2% | - | - | 0.3% |
| Singular^{®}, 5 mg tablet | 0.49% | - | - | - |

The percentage by weight of sulfoxide of formula (I) relative to montelukast sodium in the pharmaceutical compositions prepared in Examples 2 and 5 was measured by HPLC immediately after the compositions were prepared. HPLC was performed on a YMC-Pack ODS-AQ analytical column (100x4.6 mm I.D.), packed with 3µm diameter particles (YMC SEPARATION TECHNOLOGY). The mobile phase was a mixture of acetonitrile and 20 mM KH₂PO₄ at pH 2.0 (60:40) and its flow-rate was 1.0 mL/min. The UV detector was set at 285 nm and the column temperature was 30°C. The results are shown in Table 3.

**Table 3: Stability of Montelukast Sodium Compositions**

| Sample | % Sulfoxide of Formula (I) |
|---|---|
| Composition of Example 2 | 0.99 |
| Composition of Example 6 | 0.05 |

As can be seen from the data presented, the formulations manufactured by wet granulation (Examples 2 and 4) are less stable than those manufactured from a dry-mix of ingredients (Examples 3 and 5). However, in view of physical processing considerations, the preferred method of manufacture for chewable tablets was found to be wet granulation. When using wet granulation, the careful choice of excipients is particularly important so as to reduce the chance of instability.

### Example 8:

A pharmaceutical composition of montelukast sodium chewable tablets was prepared by wet granulation. A mixture was made of montelukast sodium (42.1 g), hydroxypropyl cellulose (40 g), sodium starch glycolate (96 g), mannitol granular (512 g), color iron oxide (4 g), aspartame (4 g), sodium lauryl sulfate (14.4g) and flavor (32 g). The mixture was then granulated using purified water as a granulating liquid to form a granulate. The granulate was dried, milled and blended with magnesium stearate (16 g) to form a blend. The blend was compressed into chewable tablets. Further aspects and features of the present invention are set out in the following numbered clauses.
1. A stable pharmaceutical composition comprising montelukast or a salt thereof and a pharmaceutically acceptable excipient selected from at least one of diluent, binder, disintegrant, wetting agent, lubricant, or glidant, provided that the pharmaceutically acceptable excipient is not microcrystalline cellulose.
2. The pharmaceutical composition of clause 1, wherein the composition contains the corresponding montelukast sulfoxide wherein the corresponding montelukast sulfoxide of in the composition does not increase by more than 1% by weight from the initial amount of montelukast after storage at about 40°C and about 75% relative humidity for 3 months.
3. The pharmaceutical composition of clause 2, wherein the salt is montelukast sodium and the corresponding sulfoxide is the sulfoxide of formula (I): 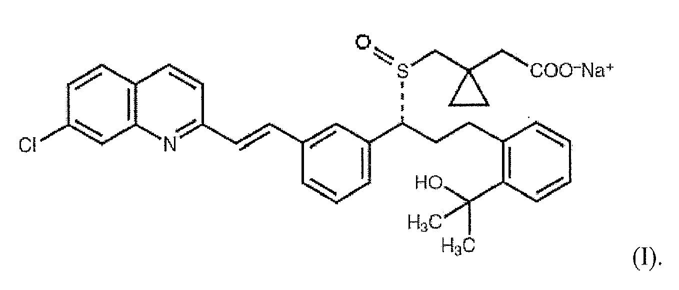
4. The pharmaceutical composition of clause 2 or clause 3, wherein the amount of the corresponding sulfoxide does not increase by more than 0.5% by weight of the initial amount of montelukast after storage at about 40°C and about 75% relative humidity for 3 months.
5. The pharmaceutical composition of clause 4, wherein the amount of the corresponding sulfoxide does not increase by more than 0.3% by weight of the initial amount of montelukast after storage at about 40°C and about 75% relative humidity for 3 months.
6. The pharmaceutical composition of clause 5, wherein the amount of the corresponding sulfoxide does not increase by more than 0.1 % by weight of the initial amount of montelukast after storage at about 40°C and about 75% relative humidity for 3 months.
7. A pharmaceutical composition comprising montelukast or a salt thereof and a pharmaceutically acceptable excipient, provided that the pharmaceutically acceptable excipient is not microcrystalline cellulose, wherein the corresponding montelukast sulfoxide is present in an amount of not more than 0.2% by weight of montelukast or a salt thereof immediately after preparation.
8. The pharmaceutical composition of clause 7, wherein the corresponding sulfoxide is present in an amount of not more than 0.1 % by weight of montelukast immediately after preparation.
9. The pharmaceutical composition of clause 7 or clause 8, wherein the salt is montelukast sodium and the corresponding sulfoxide is the sulfoxide of formula (I): 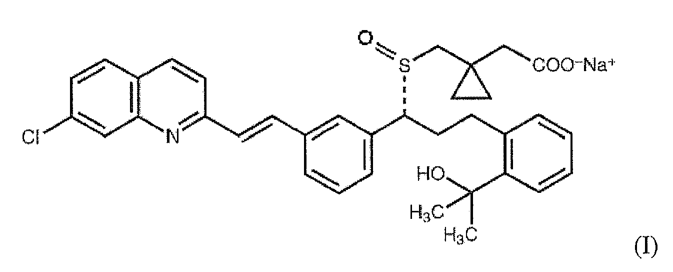
10. The pharmaceutical composition of any one of the preceding clauses, prepared by direct compression from a dry mix.
11. The pharmaceutical composition of any one of clauses 1 to 10, prepared by wet granulation.
12. The pharmaceutical composition of any one of the preceding clauses, wherein the composition is in a solid dosage form.
13. The pharmaceutical composition of clause 12, wherein the solid dosage form is a tablet or a capsule.
14. The pharmaceutical composition of clause 13, wherein the solid dosage form is a film coated tablet further comprising a coating agent, provided that the coating agent is not microcrystalline cellulose.
15. The pharmaceutical composition of clause 14, comprising montelukast sodium, lactose monohydrate, hydroxypropylcellulose, starch, sodium starch glycolate and magnesium stearate.
16. The pharmaceutical composition of clause 15, wherein the montelukast sodium is present in an amount of about 5% by weight, the lactose monohydrate is present in an amount of about 62% by weight, the hydroxypropylcellulose is present in an amount of about 2% by weight, the starch is present in an amount of about 18% by weight, the sodium starch glycolate is present in an amount of about 9% by weight, the magnesium stearate is present in an amount of about 1% by weight, and the coating agent is present in an amount of about 3% by weight.
17. The pharmaceutical composition of clause 14, comprising montelukast sodium, lactose monohydrate, hydroxypropylcellulose, starch, sodium lauryl sulfate, sodium starch glycolate and magnesium stearate.
18. The pharmaceutical composition of clause 13, wherein the tablet is a chewable tablet further comprising at least one additional pharmaceutically acceptable excipient selected from at least one of sweetening agent, flavoring agent, or coloring agent, provided that the additional pharmaceutically acceptable excipient is not microcrystalline cellulose.
19. The pharmaceutical composition of clause 18 comprising montelukast sodium, hydroxypropylcellulose, sodium starch glycolate, mannitol, color iron oxide, aspartame, flavoring agent, and magnesium stearate.
20. The pharmaceutical composition of clause 19, wherein the montelukast sodium is present in an amount of about 2% by weight, hydroxypropylcellulose is present in an amount of about 2% by weight, sodium starch glycolate is present in an amount of about 5% by weight, mannitol is present in an amount of about 87% by weight, color iron oxide is present in an amount of about 0.5% by weight, aspartame is present in an amount of about 0.5% by weight, flavoring agent is present in an amount of about 2% by weight, and magnesium stearate is present in an amount of about 1% by weight.
21. A pharmaceutical composition of clause 18 comprising montelukast sodium, hydroxypropylcellulose, sodium starch glycolate, mannitol, color iron oxide, aspartame, sodium lauryl sulfate, flavoring agent and magnesium stearate
22. A process for preparing a pharmaceutical composition as defined in any one of the preceding claims, comprising combining montelukast or a pharmaceutically acceptable salt thereof with a pharmaceutically acceptable excipient selected from at least one of diluent, disintegrant, wetting agent, lubricant, or glidant, provided that the pharmaceutically acceptable excipient is not microcrystalline cellulose.
23. The process of clause 22, wherein each of the pharmaceutically acceptable excipients is first tested in order to assess its suitability in a stable Montelukast pharmaceutical composition.
24. The process of clause 20 or clause 21, wherein the pharmaceutical composition is prepared by wet granulation.
25. The process of clause 22 for preparing a pharmaceutical composition as defined in clause 17 comprising blending montelukast sodium, lactose monohydrate, hydroxypropylcellulose, starch, sodium lauryl sulfate, sodium starch glycolate and magnesium stearate in a dry mixing method and compressing the blended components into tablet form.
26. The process of clause 22 for preparing a pharmaceutical composition as defined in clause 21 comprising preparing a mixture of montelukast sodium, hydroxypropylcellulose, sodium starch glycolate, mannitol, color iron oxide, aspartame, sodium lauryl sulfate and flavoring agent; granulating the mixture in a wet granulation method; blending the granulated mixture with magnesium stearate to form a blend; and compressing the blend into a chewable tablet.

## Claims

1. A pharmaceutical composition of montelukast comprising montelukast or a salt thereof and at least one wetting agent.

2. The pharmaceutical composition of claim 1, wherein the wetting agent is sodium lauryl sulfate.

3. The pharmaceutical composition of any preceding claim further comprising at least one pharmaceutically acceptable excipient.

4. The pharmaceutical composition of claim 3, wherein the pharmaceutically acceptable excipient is chosen by:
a) providing a mixture of montelukast and a pharmaceutically acceptable excipient wherein the pharmaceutically acceptable excipient is present in an amount that does not increase the amount of the corresponding sulfoxide of montelukast in the composition to exceed 1% by weight of the initial amount of montelukast after the composition has been stored at about 55 °C for 48 hours;
b) storing the mixture at 55 °C for 48 hours;
c) determining the presence and amount of a montelukast sulfoxide in the mixture;
and
d) selecting the pharmaceutically acceptable excipient that does not increase the amount of the corresponding sulfoxide of montelukast in the mixture to exceed 1% by weight of the initial amount of montelukast in the mixture.

5. The pharmaceutical composition of claim 4, wherein the composition contains the corresponding montelukast sulfoxide wherein the corresponding montelukast sulfoxide of in the composition does not increase by more than 1% by weight from the initial amount of montelukast after storage at about 40°C and about 75% relative humidity for 3 months.

6. The pharmaceutical composition of claim 5, wherein the salt is montelukast sodium and the corresponding sulfoxide is the sulfoxide of formula (I):

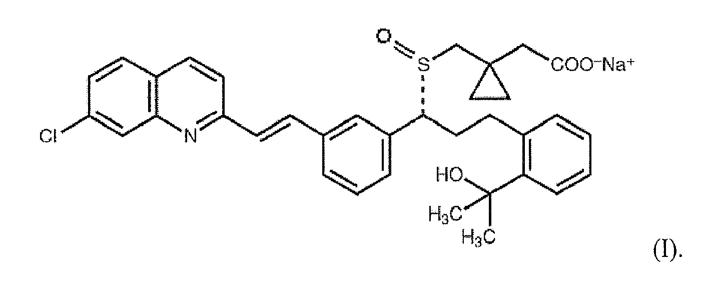

7. The pharmaceutical composition of claim 5 or claim 6, wherein the amount of the corresponding sulfoxide does not increase by more than 0.5% by weight, preferably more than 0.3% by weight, preferably more than 0.1% by weight of the initial amount of montelukast after storage at about 40°C and about 75% relative humidity for 3 months.

8. The pharmaceutical composition of any one of claims 1 to 7, prepared by wet granulation.

9. The pharmaceutical composition of any one of claims 1 to 7, prepared by direct compression from a dry mix.

10. The pharmaceutical composition of any one of the preceding claims, wherein the composition is in a solid dosage form.

11. The pharmaceutical composition of claim 10, wherein the solid dosage form is a tablet or a capsule.

12. The pharmaceutical composition of claim 10, wherein the solid dosage form is a film coated tablet further comprising a coating agent, provided that the coating agent is not microcrystalline cellulose.

13. The pharmaceutical composition of claim 10, wherein the solid dosage form is a chewable tablet.

14. The pharmaceutical composition of claim 13 comprising montelukast sodium, hydroxypropylcellulose, sodium starch glycolate, mannitol, color iron oxide, aspartame, flavoring agent, and magnesium stearate.

15. The pharmaceutical composition of claim 14, wherein the montelukast sodium is present in an amount of about 2% by weight, hydroxypropylcellulose is present in an amount of about 2% by weight, sodium starch glycolate is present in an amount of about 5% by weight, mannitol is present in an amount of about 87% by weight, color iron oxide is present in an amount of about 0.5% by weight, aspartame is present in an amount of about 0.5% by weight, flavoring agent is present in an amount of about 2% by weight, and magnesium stearate is present in an amount of about 1% by weight.
